# EUROPEAN PATENT SPECIFICATION

(11) **EP 4 623 963 B1**
(45) Date of publication and mention of the grant of the patent: **22.04.2026**
(21) Application number: 24315109.9
(22) Date of filing: 28.03.2024
(51) Int. Cl.: A61M 5/315

(54) **ELECTRONIC ADD-ON MODULE AND ASSEMBLY OF AN ELECTRONIC ADD-ON MOD-ULE AND A DRUG DELIVERY DEVICE**
ELEKTRONISCHES ZUSATZMODUL UND ANORDNUNG EINES ELEKTRONISCHEN ZUSATZMODULS UND EINER ARZNEIMITTELABGABEVORRICHTUNG
MODULE SUPPLÉMENTAIRE ÉLECTRONIQUE ET ENSEMBLE D'UN MODULE SUPPLÉMENTAIRE ÉLECTRONIQUE ET DISPOSITIF D'ADMINISTRATION DE MÉDICAMENT

(43) Date of publication of application: 01.10.2025
(73) Proprietor: Sanofi, 75017 Paris (FR)
(72) Inventor: DRAPER, Paul Richard, CV34 4AB Warwick Warwickshire (GB); O'HARE, Aidan Michael, CV34 4AB Warwick Warwickshire (GB); VEASEY, Robert Frederick, CV34 4AB Warwick Warwickshire (GB); WALLACE, Andrew, CV34 4AB Warwick Warwickshire (GB)
(74) Representative: Keil & Schaafhausen Patentanwälte PartGmbB

(56) References cited:
- EP-A1- 3 430 981
- US-A1- 2016 235 925
- US-A1- 2023 135 526
- US-A1- 2023 338 666
- US-B2- 10 258 745

## Description

The present disclosure is generally directed to an electronic add-on module and to an assembly of an electronic system, e.g. an electronic add-on module, which is configured to be releasably attached to a drug delivery device.

Electronic add-on modules for releasable attachment to drug delivery devices are generally known and often used to provide further functionalities to drug delivery devices. As such for example electronic add-on modules are known which measure relevant data with respect to dose setting and/or dose dispensing. An exemplary data collection device for attachment to an injection device is for example shown in WO 2016/198516 A1. Further, modules for attachment to an injection device are known from EP 3 430 981 A1, US 2016/235925 A1, US 2023/135526 A1 and US 10 258 745 B2.

Furthermore, a large number of different drug delivery devices are known, some of which make a noise, e.g. a click sound, during dose setting and/or dose dispensing, the start of dose dispensing and/or the end of dose dispensing. A drug delivery device with a clicker arrangement providing a click sound at the end of dose dispensing is known, for example, from EP 3 164 173 A1. The use of sounds that indicate a dose event, e.g. the start of a dose delivery, can be an important indication, particularly for visually handicapped users. However, sound feedback can also be helpful for unexperienced users to recognize correct operation of the drug delivery device.

If an electronic add-on module is used with a drug delivery device, e.g. for monitoring or measuring dose events, it is desirable if the electronic add-on module acts independently and safe and, for example, reliably records the amount of dose dispensed or the time at which a dose is dispensed. Often sensors are used for monitoring and measuring, for example, sensors for optical detection of relative movements. However, these sensors are typically complicated to implement. In addition, relative movements are not always decisive and indicative of an actual drug delivery device event. For example, a brief interruption of a dose delivery does not necessarily mean that dose dispensing has ended, however, may as such be recognized by an electronic add-on module which relies only on, for example, optical sensing, i.e. optical detection of relative movements.

Further, using the detection of relative movements often requires further, especially structural, modifications to the drug delivery device as movements of components inside the drug delivery device may best to be sensed in order to gather relevant dose event information. These structural modifications, for example the provision of encoder patterns, are less suitable for retrofitting a drug delivery device with an electronic add-on module.

Consequently, there is a need to provide additional means to ensure reliable and safe monitoring and measurement of a drug delivery device with an electronic add-on module, wherein no or only limited structural modifications of the drug delivery devices are required. Hence, it is an object of the present invention to provide an improved electronic add-on module that is configured to detect dose events of a drug delivery device reliably and safe.

This object is solved by an electronic add-on module according to claim 1 as well as by an assembly according to claim 14.

The electronic add-on module may be releasably attached to the drug delivery device by fastening means for releasable attachment, for example, interacting mechanical coupling elements or by frictional or elastic engagement. An assembly comprises a drug delivery device and an electronic add-on module configured for releasable attachment to the drug delivery device.

The electronic add-on module comprises at least a housing with an electric power source arrange inside the housing. The electric power source may be a battery, for example a coin cell. The housing may comprise the aforementioned fastening means for releasable attachment to a drug delivery device. The electronic add-on module may be configured to be releasably attached to a dose button of a drug delivery device. The electronic add-on module may be configured to be releasably attached to a dial grip of a drug delivery device. The housing may comprise several parts or a single part, e.g. an injection-molded plastic part. The electronic add-on module also has a circuit board assembly which is electrically connected to the electric power source. The circuit board assembly may comprise a printed circuit board assembly (PCB). The circuit board assembly may comprise a substrate equipped with electronic components. Electronic components may be integrated circuits, processors, conductors, wireless modules or the like. The electronic components may be electrically connected to the circuit board assembly and may therefore also be supplied by power of the electric power source. The circuit board assembly may be arranged inside the housing and between the electric power source and an attachment portion for releasable attachment to a drug delivery device.

Further, an acoustic sensor arrangement forms part of the electronic add-on module, wherein the acoustic sensor arrangement is electrically connected to the circuit board assembly. The acoustic sensor arrangement is configured to detect noises and convert them into a signal, which may be indicated, for example, by a voltage. The acoustic sensor arrangement may thus comprise a housing in which an acoustic sensor may be arranged. **In** principle, the acoustic sensor arrangement is configured to detect both background noises, i.e. noises that do not emanate from the drug delivery device, the electronic add-on module or another supplementary device, and to output a corresponding first output signal, as well as noises that emanate from the drug delivery device, the electronic add-on module or another supplementary device. Background noises may also be called non-relevant noises or sounds which may be responsible for non-relevant first output signals of the sensor arrangement. These non-relevant first output signals or background noises may not be generated by dose events but still be detected by the acoustic sensor arrangement. Noises or sounds other than background noises may be called relevant noises. These noises or sounds are indicative for dose events. The terms "noise" and "sound" are interchangeably used herein. For example, the acoustic sensor arrangement may be configured to detect click sounds from the drug delivery device or may be configured to detect beeps (beep sounds) emitted by the drug delivery device or a supplementary device such as a mobile phone, for example. The sounds may be generated mechanically, electromechanically or electronically, e.g. by a physical impact, the conversion of a mechanical contact into an electronic signal or by a purely electronic signal. The acoustic sensor arrangement may be a microphone or may comprise a microphone.

The sensor arrangement may provide a digital or analog first output signal. The sensor arrangement may include a first amplifier stage, a circuit to generate an electrical bias voltage for the sensor, a circuit to generate a digital output or some other electronic circuitry required to obtain this first output signal. The first output signal is the output of the sensor arrangement, which includes the sensor itself and any electronic circuitry either integrated into the sensor or required to obtain a usable electrical signal from the sensor.

Further, the electronic add-on module comprises a processing circuit configured to process the first output signal from the acoustic sensor arrangement. In other words, the electronic add-on module comprises the processing circuit that receives and processes the first output signal that is output from the acoustic sensor arrangement due to a non-relevant sound or a relevant sound. The processing circuit may comprise a central processing/computing unit, an analog-to-digital converter, storage etc.

If, for example, the first output signal comprises a voltage pulse, a magnitude of the pulse, i.e. the amplitudes, and a signal duration, for example, may be used to determine whether the noise was a relevant or a non-relevant signal. The volage pulse may thus comprise positive and negative voltages. The voltage pulse may be a transient voltage pulse. Based on the first output signal received from the acoustic sensor arrangement, the processing circuit is therefore configured to detect a dose event such as a dose dialing event, a dose dispensing event and/or a dose amount, dialed and/or dispensed. For example, the processing circuit may allow to determine a start and an end of a dose dispensing event based on an indicative click sound detected by the acoustic sensor arrangement at the start and at the end of the dose dispensing. If for example, the acoustic sensor arrangement only detects a signal, which may be indicative of a click sound at the start of the dose dispensing but a further first output signal at the end of dose dispensing is missing, the electronic add-on module may not register this event as a dose dispensing event. In this case, the user may not have fully dispensed the dose. The electronic add-on module may also be able to determine the number of recurring sounds so that the electronic add-on module may use these sounds to draw conclusions about a set amount of dose or a dispensed amount of dose. For example, a click sound may be emitted for each unit of medication when setting the dose. It is also possible for a corresponding sound to be emitted for each unit of medication when the dose is dispensed.

The aforementioned possibilities for using the first output signal are only an excerpt of possibilities for using the first output signal. However, there are many other ways of using the first output signal for detection of dose events, which is why the present invention is not limited to the specific type of signal use for detection of the specific event.

The detection of a noise or an (acoustic) sound, e.g. click sounds from a drug delivery device, allows for an alternative use of provided features for the detection of dose events. This means that in addition, for example, to the detection of movements, acoustic signals may also be detected. As the sound waves emerge from the device, the detection of these features is minimally invasive. As the sound features regularly provide important feedback for unexperienced or handicapped users, these sound features must be reliable and highly precise. This means that a sound is only emitted if, for example, a dose amount has actually been increased. Consequently, drug delivery devices typically provide a clean and distinct sound, for example, during dose dispensing, wherein one click represents the dispense of one unit. The sound feature therefore provides extremely precise information that can be recorded using the disclosure and used for data processing, for example.

Hence, the disclosure allows, for example, for analog acoustic sensing by producing peaks, for example, voltage peaks that can be processed, for example, counted, to identify dose events. According to one aspect, the sound signal may be used to confirm a dose event.

In one aspect, the acoustic sensor arrangement may be coupled to the circuit board assembly. For example, the acoustic sensor arrangement may be arranged on the circuit board assembly or may be hinged thereto. Further, the acoustic sensor arrangement may comprise a port hole that allows sound waves to enter the acoustic sensor arrangement. In other words, the acoustic sensor arrangement may be ported. The port hole may be a hole for transmission of soundwaves towards a sensor inside the senor arrangement. The port hole may be comparatively small with respect to the overall acoustic sensor arrangement. For example, the port hole may comprise a diameter between 0.1 mm and 0.7 mm, for example, of about 0.25 mm. The acoustic sensor arrangement may have a cuboid shape with outer edge lengths of between 1 mm and 4 mm. For example, the length of the acoustic sensor arrangement may be approximately 3.0 mm, the width approximately 2.0 mm and the height approximately 1 mm. Further, the port hole may be arranged so that it is not covered, when the electronic add-on module is releasably attached to a drug delivery device. Providing the acoustic sensor inside the acoustic sensor arrangement may protect the sensor. For example, the acoustic sensor arrangement may be protected against ingress of dirt and/or liquid.

In one aspect, the electronic add-on module may have a coupling portion for releasable attachment to a drug delivery device, and wherein the port hole is directed towards the coupling portion. In other words, when the electronic add-on module is attached to a drug delivery device, the port hole may be arranged to face the drug delivery device. When the coupling portion is releasably attached to a dose dial grip of the drug delivery device, wherein the drug delivery device comprises a needle at its distal end, the port hole may (substantially) face in a distal direction, i.e. towards the distal end of the drug delivery device. This arrangement may be particularly helpful, if the relevant sound, i.e. the sound of interest, comes from the drug delivery device as the port hole may then be directed towards a sound source which may maximize sensitivity to the sound from the drug delivery device. If the sound source may for example be inside the electronic add-on module, however, it may be advantageous if the port hole would face this sound source. Further, in one aspect, when the electronic add-on module is releasably attached to the drug delivery device, the port hole may be arranged closer to the drug delivery device than to an opposite end of the electronic add-on module facing away from the drug delivery device. This may additionally increase sensitivity of the acoustic sensor arrangement as it may be arranged closer to a sound source of the drug delivery device. A distance between the port hole and a sound source of the drug delivery device may be less than 150 mm, for example, between 10 mm and 50 mm. However, since in addition to the distance and the orientation of the port hole, a frequency of a sound to be detected and layers of material between the sound source and the acoustic sensor arrangement are also decisive in order to comprise sufficient sensitivity to detect a sound of a sound source, the distances given are only reference values and the distance may also be greater than 150 mm or less than 10 mm.

In a further aspect, the circuit board assembly, when the electronic add-on module is releasably attached to the drug delivery device, may be arranged between the drug delivery device and the acoustic sensor arrangement. The acoustic sensor arrangement may thus be shielded from the coupling portion by the circuit board assembly, which may provide additional protection for the acoustic sensor arrangement, for example additional protection against ingress of dirt or liquid. In one aspect, the acoustic sensor arrangement may be directly coupled to a side of the circuit board assembly facing away from the coupling portion. In order not to cover the port hole by the circuit board assembly and/or to increase sensitivity of the acoustic sensor arrangement, the circuit board assembly may comprise a through hole configured to directly guide sound waves to the port hole of the acoustic sensor arrangement. Arrangement of the port hole facing away from the circuit board assembly may be called "top-ported", wherein arrangement of the port hole facing the circuit board assembly may be called "bottom-ported". The present invention allows for both, a "top-ported" as well as a "bottom-ported" arrangement of the port hole.

According to a further aspect, the processing circuit may be configured to compare the first output signal from the acoustic sensor arrangement to a threshold value, for example a time threshold value or an amplitude threshold value. Further, detection by the processing circuit, i.e. detection of a dose dialing event, a dose dispensing event and/or a dose amount, dialed and/or dispensed, may be triggered based on the comparison with the threshold value. The design of the acoustic sensor arrangement will be such that the quiescent voltage output is below the threshold value and detected events cause it to rise above the threshold value. However, in some implementations, this may be inverted so the quiescent voltage output of the acoustic sensor arrangement is above the threshold value and detected events cause it to fall below the threshold value. "Comparison" may mean that the processing circuit may identify a transition of a voltage of the first output signal from below to above the threshold value. Alternatively, the processing circuit may identify a transition of a voltage of the first output signal from above to below the threshold value. **In** other words, the processing circuit may identify if an amplitude of the first output signal is higher (greater) or smaller (lower) than a threshold value. However, the processing circuit may also identify a number of transitions between an area below the threshold value and an area above the threshold value, i.e. a number of changes at which the first output signal exceeds the threshold value and falls below the threshold value. Alternatively, the processing circuit may also identify a number of transitions between an area above the threshold value and an area below the threshold value. The use of a threshold value may ensure that not every background noise is interpreted as being relevant. For example, a noise caused by picking up the drug delivery device may not be sufficient to generate a first output signal that crosses the threshold value. The noise of picking up would therefore be background noise, which would not be considered relevant and would not be used for the detection of dose events. Furthermore, although the amplitude of a background noise may exceed the threshold value, it is possible that a change below and above the threshold value, which may be significant to be interpreted as a relevant noise, does not take place sufficiently often or takes place too often, which is why the background noise may be classified as non-relevant. For an alternating pulse signal, the threshold value may be selected above or below a zero crossing. For example, the threshold value may be chosen to be between 10% and 50%, e.g. 20%, above or below the zero crossing in relation to a maximum expected amplitude of a first output signal of a relevant sound, so that pulses that do not cross the threshold value are not used for detection. This means that if a maximum expected amplitude of a relevant sound is +400 mV, the threshold value for a case where it is chosen to be 10% above the zero crossing is +40 mV. Consequently, only pulses that do cross the threshold value or that do change between an area below the zero crossing and above the zero crossing a predetermined number of times are used for detection.

In one aspect, the processing circuit may comprise an analog-to-digital converter configured to convert the first output signal to a numerical value. For example, the analog-to-digital converter may convert a voltage output signal of the acoustic sensor arrangement into a numerical value. Further, the processing circuit may be configured to compare the numerical value to a numerical threshold value.

Further, the electronic add-on module comprises, in addition to the processing circuit, a conditioning circuit configured to condition the first output signal of the acoustic sensor arrangement. In other words, the conditioning circuit may modulate the first output signal received from the acoustic sensor arrangement. The first output signal, when received by the conditioning circuit may thus be untreated, unconditioned or unprocessed and may be described as a "raw signal". After conditioning, the signal may then be referred to as a "conditioned signal". The conditioning of the first output signal is performed prior to being processed in the processing circuit, i.e. before the processing, for example, detection takes place. In other words, detection by the processing circuit may be performed based on a conditioned signal, i.e. a conditioned first output signal. The process circuit may thus be downstream compared to the conditioning circuit. "Downstream" may mean that something, i.e. a circuit or an electronic component, is located further away from the acoustic sensor arrangement in the signal path, wherein "upstream" may mean that something, i.e. a circuit or an electronic component, is located closer to the acoustic sensor arrangement in the signal path. For example, as a signal enters the conditioning circuit before it enters the processing circuit, the conditioning circuit is upstream compared to the processing circuit, wherein the processing circuit is downstream compared to the conditioning circuit. "Upstream" and "downstream" do not refer to items being necessarily physically closer or further away in proximity to the acoustic sensor, but to them being schematically closer or further away in the signal path.

In order to keep the electronics and software architectures simple, low cost and small, according to one aspect the conditioning circuit and/or the processing circuit are predominantly provided by analog electronic components.

The conditioning of the first output signal comprises at least one of, several of or all of the following conditioning operations:
- removing a DC voltage;
- limiting a maximum current running in the conditioning circuit;
- amplifying the first output signal by increasing an amplitude of the first output signal; and
- modulating the first output signal by elongating a duration the first output signal, rectifying the first output signal and/or smoothing the first output signal.

"Removing a DC voltage" may be understood as the removal of a phantom power supply or a DC bias voltage of the acoustic sensor arrangement. Typically, acoustic sensor arrangements, i.e. also microphones, have a DC bias voltage in order to be able to react to incoming acoustic sounds, for example by generating an electric field through the bias voltage. A state in which no sound is detected by the acoustic sensor arrangement may be a "quiescent state". The sound subsequently detected by the acoustic sensor arrangement may lead to the pulse described above, i.e. an alternating voltage or an alternating current. As the DC bias voltage may vary depending on the specific acoustic sensor arrangement used, removing or filtering out the DC bias voltage may allow to use processing regardless of the specific implemented acoustic sensor arrangement. Further, a non-zero quiescent state may reduce the dynamic range of the processing circuit and may thus reduce the efficacy of the threshold value detection. Removing or filtering therefore means that the DC bias voltage does not affect the processing circuit, i.e. the DC bias voltage may enter the conditioning circuit but does not form part of the conditioned signal.

"Limiting a maximum current running in the conditioning circuit" may ensure that voltages outside a specified absolute maximum voltage range do not pass the conditioning circuit. Consequently, it is not possible for such voltages to damage downstream components or the downstream processing circuit.

"Amplifying the first output signal by increasing an amplitude of the first output signal" may allow to use the first output signal in a (digital)logic circuit. The first output signal of the acoustic sensor arrangement corresponding, for example to a dose click, may have a smaller amplitude than an amplitude that may be detectable by typical digital logic circuits. **If** the first output signal is processed by an analog-to-digital converter, the first output signal would require a lower reference voltage for analog to digital conversion than is typical required for an embedded microcontroller. Increasing the signal amplitude may also increase the signal to noise ratio. Amplification may thus increase the magnitude of the signal, for example a time-varying voltage or current.

"Modulating the first output signal by elongating a duration the first output signal, rectifying the first output signal and/or smoothing the first output signal" may allow for better processing of the first output signal. Typically, a duration of a first output signal corresponding to a click sound, for example a dose click sound provided during dose dialing, is relatively short in time. Typically, less than 3 milliseconds (ms). Therefore, fast operation of the processing circuit may be required to detect the signal. Elongating the first output signal may thus allow for the processing circuit to operate slower. Further, a sound, for example a click sound, detected by the acoustic sensor arrangement may be a damped sine wave which may cross the threshold value, i.e. change between values below and above the threshold value, multiple times as described above. Accordingly, it may be difficult to differentiate between consecutive click sounds provided by consecutive dose clicks and a single dose click. Therefore, rectifying the first output signal may allow to limit a number of times a first output signal of a single click sound may cross or transit a threshold value. Hence, differentiating between a first output signal received from a single sound, for example a single dose click, compared to a first output signal received from multiple sounds, for example multiple dose clicks, may be easier.

In one aspect, the conditioning circuit may thus comprise at least one of, several of or all of the following components and functionalities:
- a DC-filter unit configured to remove a DC bias voltage of the acoustic sensor arrangement;
- an electrical impedance unit configured to limit a current into an amplifier unit;
- the amplifier unit configured to increase an amplitude of the first output signal (...);
- an amplifier-decoupling unit configured to decouple a power supply to the amplifier unit; and
- an envelope detection circuit configured to modulate the first output signal.

The DC-filter unit may be provided by a capacitor in a circuit path that keeps out the DC voltage.

The electrical impedance unit may be provided by a resistor. By setting the resistance value of the resistor, the current flow into the amplifier unit may be limited, i.e. the maximum current flowing into the amplifier unit. The amplifier unit may thus be protected from potential damage.

**In** order to make the first output signal more suitable for detection and processing by the processing circuit, the amplifier unit may be provided by an amplifier used for increasing a magnitude of the signal, for example by setting a gain and increasing an amplitude of the first output signal.

An amplifier-decoupling unit may be provided by a decoupling capacitor, also known as a bypass capacitor. The amplifier-decoupling unit may thus reduce transmission of noise (signal noise) present on the electric power source to the conditioning circuit output.

Further, the envelope detection circuit may elongate the duration of the first output signal as aforementioned. **In** other words, the envelope conditioning circuit may be configured to comprise a fast response to an incoming first output signal, for example a rising voltage, and a slow response afterwards, i.e. to falling voltages. The processing circuit may thus be allowed to be slower. The envelope detection circuit may thus elongate, rectify and/or smooth the signal.

Without conditioning the first output signal, for example by the envelope conditioning circuit, it may be difficult to precisely process sound signals of drug delivery devices comprising for example two clicker arms as two click sounds may be very close, for example separated by only approximately 1 ms.

**In** one aspect, the envelope detection circuit may be connected downstream of the amplifier unit and electrically connected to the processing circuit. **In** other words, the first output signal must first pass the amplifier unit to reach the envelope detection circuit and is then directly passed on to the processing circuit after the conditioned first output signal leaves the envelope detection circuit. Additionally or alternatively, the DC-filter unit and/or the electrical impedance unit may be connected upstream of the amplifier unit. For example, the DC-filter unit may be arranged upstream with respect to the low-pass filter unit, the electrical impedance unit may be arranged upstream with respect to the amplifier unit, and the amplifier unit may be arranged upstream with respect to the envelope conditioning circuit. This may allow for improved conditioning of the first output signal provided to the conditioning circuit. Therefore, detection by means of the first output signal may be improved.

**In** one aspect, the envelope detection circuit may comprise an RC element with a resistor and a grounded capacitor, and a diode connected in parallel with the resistor. For example, the envelope detector may either have a resistor, it may have a grounded capacitor and it may have a diode in parallel with a resistor, or it may have a RC element with a resistor, a grounded capacitor and a diode in parallel with the resistor. A voltage may thus directly pass through the diode in order to charge the capacitor. The capacitor is thus charged quickly which leads to the aforementioned fast response to rising voltages. However, the discharge of the capacitor is performed via the resistor. Consequently, the envelope detection circuit allows for a slow response to falling voltages. Therefore, when a high voltage is received, the capacitor may be charged quickly. A lower voltage afterwards may not pass the diode resulting in a delay, stretching or elongating of the signal that may then be used in the processing circuit. Hence, the envelope detection circuit allows for a slower response speed of the processing circuit since a first output of a sound, for example a click sound, may be longer in time, i.e. elongated. The envelope detection circuit may also allow for partial deletion or removal of several discrete amplitudes of a single sound, for example a single click sound.

**In** one aspect, the electronic add-on module may comprise a first portion defining an auxiliary dose dial grip. Rotation of the auxiliary dose dial grip may thus rotate the dose dial grip of the drug delivery device and may thereby be used for dose setting. The first portion may be configured to be releasably attached to a dose dial grip of the drug delivery device. The first portion may thus for example allow for mechanical coupling by interacting mechanical coupling elements or by frictional or elastic engagement. Further, the first portion may comprise a longitudinal axis. The first portion may extend along the longitudinal axis. When the electronic add-on module is releasably attached to a drug delivery device, the longitudinal axis may be in line with a longitudinal axis of the drug delivery device. Furthermore, the electronic add-on module may comprise a second portion coupled to the first portion. The coupling between the first portion and the second portion may allow the second portion to be relatively moved rotationally about the longitudinal axis and axial parallel to the longitudinal axis with respect to the first portion. **In** other words, the coupling may allow relative rotational movement about the longitudinal axis and relative axial movement parallel to the longitudinal axis with respect to the first portion. Further, the second portion may define an auxiliary dose button configured to apply pressure onto the dose button of the drug delivery device when the electronic add-on module is releasably attached to the drug delivery device.

Although described here as a two-portioned electronic add-on module, the electronic add-on module may also only comprise one part (, i.e., one portion) and, for example, only allow the triggering of a dose button of a drug delivery device, whereby a sound is generated.

Further, the electronic add-on module may additionally comprise a switch, for example a microswitch, wherein the switch may be configured to be actuated to activate electronic functionalities of the electronic add-on module. For example, the switch may be used to activate the acoustic sensor arrangement. Consequently, sound detection by the acoustic sensor arrangement may only be conducted, when the acoustic sensor arrangement is activated. Therefore, the switch may allow to reduce power consumption of electronics. In addition, the switch may prevent that sounds are detected accidentally as relevant noises although the electronic add-on module is not used. Further, at least when the electronic add-on module is releasably attached to a drug delivery device, an axial relative movement of the second portion along the longitudinal axis with respect to the first portion may actuate the switch. This relative axial movement may be used to press a dose button of the drug delivery device, wherein pressing the dose button may cause a dose to be dispensed. Consequently, the switch may only be actuated, when a dose is dispensed. If the additional electronic module has other sensors or electronic components in addition to the acoustic sensor arrangement, such as a display, these components may be activated or deactivated independently of the switch, for example by a second switch. This means that the switch may also only activate the acoustic sensor arrangement and the conditioning circuit and/or the processing circuit.

According to one aspect, the electronic add-on module may additionally comprise a clutch mechanism, wherein, when the clutch is engaged, relative rotational movement about the longitudinal axis between the first portion and the second portion may be prevented. Further, when the clutch is disengaged, relative rotational movement about the longitudinal axis between the first portion and the second portion may be allowed,

Furthermore, when the electronic add-on module is releasably attached to a drug delivery device, the switch may be configured to be actuated prior to disengagement of the clutch. Consequently, the clutch mechanism may keep the first portion and the second portion rotationally fixed, i.e. no or at least no substantial relative rotational movement between the first portion and the second portion is possible, until sufficient load is applied. Considering releasable attachment of the electronic add-on module to a dose dial grip of a drug delivery device, wherein the dose dial grip may rotate during dose dispensing, the clutch mechanism may prevent dose dispensing before disengagement of the clutch mechanism. Therefore, the switch may be activated before the clutch disengages, which may allow to actuate the switch before a dose dispensing starts. Thus, this may ensure that the acoustic sensor arrangement is activated during dose dispensing, which may allow to ensure detection of a start of a dose dispensing event or an end of a dose dispensing event.

In one aspect, the electronic add-on module may comprise at least one non-acoustic sensor arrangement. In other words, the electronic add-on module may comprise at least one further sensor arrangement which is not an acoustic sensor arrangement. The non-acoustic sensor arrangement may be an optical, a magnetic, a mechanical or a capacitive sensor arrangement. A second output signal of the at least one non-acoustic sensor arrangement may be used to classify a detected dose event into a relevant or a non-relevant dose event. For example, the processing circuit may detect, based on a first output signal from the acoustic sensor arrangement, that a dose dispensing event may have occurred, however, an optical sensor arrangement may detect no relative movement, for example no relative rotational movement between the dose dial grip with respect to the dose button, and may therefore classify the detected dose event, which was detected based on the first output signal, as a non-relevant dose event. Consequently, no dose dispensing event may be registered in a memory or sent to a further device, such as a mobile phone or the like. Using multiple sensor arrangements may thus increase accuracy of the electronic add-on module. For example, using multiple sensor arrangements may thus prevent a user to dispense a dose, which would otherwise not be required. Further, it may avoid external influences affecting the accuracy of the electronic add-on module.

Further, in one aspect, the acoustic sensor arrangement may be a MEMS (micro-electromechanical systems) acoustic sensor arrangement, for example a capacitive or piezoelectric MEMS acoustic sensor arrangement. The acoustic sensor arrangement may thus comprise a microphone which is MEMS. A suitable MEMS sensor arrangement may for example be AMM-3742-T-EB-R MEMS microphone provided by PUI Audio or a Ole Wolff OWMMOA-271809A-S381FANC-BP. Although other standard acoustic sensor arrangements and technologies may also be used, the small size and low power nature of MEMS acoustic sensor arrangements make them ideal for use in an electronic add-on module with limited space and a limited electric power source. In addition, MEMS acoustic sensor arrangements, for example MEMS microphones are highly sensitive.

In a further aspect, the object may be solved by an assembly comprising a drug delivery device and an electronic add-on module according to the aforementioned aspects. The electronic add-on module may be releasably attached to the drug delivery device. The drug delivery device may for example be the drug delivery device known from EP 3 164 173 A1. Other suitable working principles of drug delivery devices to be used may for example be described in EP 1 570 876 B1, EP 2 814 547B1, EP 2 890 434 B1, WO 2005/018721 A1, WO 2009/132777 A1, WO 2014/033195 A1, US 5,693,027 A, US 6,663,602 B2, US 7,241,278 B2 or US 9,937,294 B2.

If the drug delivery device has a similar working principle as in the example of WO 2004/078239 A1, during dose setting components of the drug delivery device may perform the following movements. A housing may be stationary and may be used as a reference system for the further movements of other components. A plunger may be stationary and may be guided in a housing thread. A drive sleeve may perform a helical movement, i.e. a combined axial and rotational movement, and may be in threaded engagement with the plunger. A dial grip may perform a helical movement. A dose button may be free to rotate but axially constrained to the drive sleeve. For example, the dose button may be axially retained to the drive sleeve by a clutch. An optional clutch may perform a helical movement and may couple a number sleeve to the drive sleeve. An optional clutch spring may perform an axial movement and may be guided in housing splines and may click over clutch teeth. An optional number sleeve may be permanently fixed on the dial grip and may perform a helical movement and may be guided in a housing thread. An optional last dose nut may perform a helical movement on a drive sleeve track of the drive sleeve and may be rotationally constrained to the housing. Hence, the last dose nut may perform axial movement relative to the housing and a helical movement with respect to the drive sleeve.

During dose dispensing components of the drug delivery device may perform the following movements. The housing may remain stationary as a reference system for the further movements of other components. The plunger may perform a helical movement and may be guided in the housing thread. The drive sleeve may perform a pure axial movement and may be in threaded engagement with the plunger. The dose dial grip may perform a helical movement and may be permanently fixed on the number sleeve. The dose button may perform an axial movement if coupled to the drive sleeve and/or the clutch. The optional clutch may perform pure axial movement and may de-couple the number sleeve from the drive sleeve. The optional clutch spring may perform pure axial movement and may be rotationally constrained to the clutch due to a pressure applied to the dose button. The optional number sleeve may perform a helical movement and may be guided in the housing thread. The optional last dose nut may maintain its axial position on the drive sleeve track and may be rotationally constrained to the housing.

The components responsible for dose setting, i.e. for increasing a dose to be dispensed, may thus be considered a dose dialing mechanism, wherein the components responsible for dose dispensing, i.e. for dispensing the dose, for example from the device into a user's body, may be considered a dose dispensing mechanism. The electronic add-on module may comprise an interface for a dose dialing mechanism and/or a dose dispensing mechanism. Actuation of the corresponding mechanism, i.e. the dose dialing mechanism and/or the dose dispensing mechanism, may cause a clicking sound, for example, by a deflected clicker arm which may be deflected, by ratchets or the like, due to relative movements of the respective mechanism. The electronic add-on module may be configured to detect a dose dialing event, a dose dispensing event and/or a dose amount, dialed and/or dispensed, based on a first output signal of the acoustic sensor arrangement resulting from the clicking sound, preferably provided by a clicker arm.

The beneficial technical effects described with respect to the electronic add-on module may thus also account for the assembly. Consequently, the assembly may allow for improved dose event detection.

The electronic add-on module may be an electronic dose recording system for determining, storing and/or transmitting data indicative of at least a condition of the drug delivery device or its use. For example, the system may detect if the drug delivery device is switched between a dose setting mode and a dose dispensing mode and vice versa. **In** addition or as an alternative, the system may detect if a dose is set and/or if a dose is dispensed. Still further, the system may detect the amount of dose selected and/or the amount of dose dispensed. Preferably, the electronic add-on module is configured such that it may be switched from a first state having lower energy consumption into a second state having higher energy consumption. This may be achieved by operation of the electronic add-on module, especially by actuating the microswitch. The first state may be a sleeping mode and the second mode may be a detection and/or communication mode. As an alternative, an electronic control unit may issue a command, e.g. a signal, to another unit of the electronic dose recording system such that this unit is switched on or rendered operational.

The electronic add-on module may further comprise a communication unit for communicating with another device, e.g. a wireless communications interface for communicating with another device via a wireless network such as Wi-Fi or Bluetooth, or even an interface for a wired communications link, such as a socket for receiving a Universal Series Bus (USB), mini-USB or micro-USB connector. Preferably, the electronic add-on module comprises an RF, Wi-Fi and/or Bluetooth unit as the communication unit. The communication unit may be provided as a communication interface between the electronic addon module and the exterior, such as other electronic devices, e.g. mobile phones, personal computers, laptops and so on. For example, dose data may be transmitted by the communication unit to the external device. The dose data may be used for a dose log or dose history established in the external device.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (anti-diabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide.

Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)des(B30) human insulin, B29-N-omega-carboxypentadecanoyl-gamma-L-glutamyldes(B30) human insulin (insulin degludec, Tresiba^{®}); B29-N-(N-lithocholyl-gamma-glutamyl)-des(B30) human insulin; B29-N-(w-carboxyheptadecanoyl)-des(B30) human insulin and B29-N-(w-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, small modular immunophar-maceuticals (SMIP), binding-domain immunoglobulin fusion proteins, camelized antibodies, and immunoglobulin single variable domains. Additional examples of antigen-binding antibody fragments are known in the art.

The term "immunoglobulin single variable domain" (ISV), interchangeably used with "single variable domain", defines immunoglobulin molecules wherein the antigen binding site is present on, and formed by, a single immunoglobulin domain. As such, immunoglobulin single variable domains are capable of specifically binding to an epitope of the antigen without pairing with an additional immunoglobulin variable domain. The binding site of an immunoglobulin single variable domain is formed by a single heavy chain variable domain (VH domain or VHH domain) or a single light chain variable domain (VL domain). Hence, the antigen binding site of an immunoglobulin single variable domain is formed by no more than three CDRs.

An immunoglobulin single variable domain (ISV) can be a heavy chain ISV, such as a VH (derived from a conventional four-chain antibody), or VHH (derived from a heavy-chain antibody), including a camelized VH or humanized VHH. For example, the immunoglobulin single variable domain may be a (single) domain antibody, a "dAb" or dAb or a Nanobody^{®} ISV (such as a VHH, including a humanized VHH or camelized VH) or a suitable fragment thereof. [Note: Nanobody^{®} is a registered trademark of Ablynx N.V.];. other single variable domains, or any suitable fragment of any one thereof.

"VHH domains", also known as VHHs, VHH antibody fragments, and VHH antibodies, have originally been described as the antigen binding immunoglobulin variable domain of "heavy chain antibodies" (i.e., of "antibodies devoid of light chains"; Hamers-Casterman et al. 1993 (Nature 363: 446-448). The term "VHH domain" has been chosen in order to distinguish these variable domains from the heavy chain variable domains that are present in conventional 4-chain antibodies (which are referred to herein as "VH domains") and from the light chain variable domains that are present in conventional 4-chain antibodies (which are referred to herein as "VL domains"). For a further description of VHH's, reference is made to the review article by Muyldermans 2001 (Reviews in Molecular Biotechnology 74: 277-302).

For the term "dAb's" and "domain antibody", reference is for example made to Ward et al. 1989 (Nature 341: 544), to Holt et al. 2003 (Trends Biotechnol. 21: 484); as well as to WO 2004/068820, WO 2006/030220, WO 2006/003388. It should also be noted that, although less preferred in the context of the present invention because they are not of mammalian origin, single variable domains can be derived from certain species of shark (for example, the so-called "IgNAR domains", see for example WO 2005/18629).

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope and spirit of the present invention, which encompass such modifications and any and all equivalents thereof.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).

As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

The terms "axial", "radial", or "circumferential" as used herein may be used with respect to a longitudinal axis of the electronic add-on module, the first portion, the second portion, the drug delivery device, the cartridge, the housing, the cartridge holder or the assembly of the drug delivery device and the electronic add-on module, e.g. the axis which extends through the proximal and distal ends of the cartridge.

"Distal" is used herein to specify directions, ends or surfaces which are arranged or are to be arranged to face or point towards dispensing end of the electronic add-on module or the drug delivery device or components thereof and/or point away from, are to be arranged to face away from or face away from the proximal end. On the other hand, "proximal" is used to specify directions, ends or surfaces which are arranged or are to be arranged to face away from or point away from the dispensing end and/or from the distal end of the electronic add-on module or the drug delivery device or components thereof. The distal end may be the end closest to the dispensing and/or furthest away from the proximal end and the proximal end may be the end furthest away from the dispensing end. A proximal surface may face away from the distal end and/or towards the proximal end. A distal surface may face towards the distal end and/or away from the proximal end. The dispensing end may be the needle end where a needle unit is or is to be mounted to the device, for example. Similarly, a distal element compared to a proximal element is located closer to the dispensing end than to the proximal end. Furthermore, when the electronic add-on module is considered alone, the term "distal" may be used with regard to the more distal end of the electronic add-on module, which is located closer to the dispensing end of the drug delivery device when attached to the drug delivery device, and the term "proximal" may be used with regard to the proximal end of the electronic add-on module, which is located further away from the dispensing end of the drug delivery device when attached to the drug delivery device.

In the following, non-limiting, examples of the electronic add-on module, the drug delivery device and the assembly of the drug delivery device and the electronic add-on module are described in more detail by making reference to the drawings, in which:
- Figure 1: shows a drug delivery device;
- Figure 2: shows a drug delivery device with an exploded view of an electronic add-on module arranged above the drug delivery device;
- Figure 3: shows an exemplary second portion of an electronic add-on module;
- Figure 4: shows a first state, prior to any movement, of a sequence of movement of an assembly;
- Figure 5: shows an intermediate state of a sequence of movement of the assembly;
- Figure 6: shows a final state of movement of a sequence of movement of the assembly;
- Figure 7: shows a circuit board assembly with a "top-ported" acoustic sensor arrangement;
- Figure 8: shows a circuit board assembly with a "bottom-ported" acoustic sensor arrangement;
- Figure 9: shows a conditioning circuit;
- Figure 10: shows an unconditioned first output signal;
- Figure 11: shows a first output signal and a conditioned first output signal.

In the Figures, identical elements and components as well as identical elements and components in different examples or embodiments, i.e. elements and components acting identical or provided for the same purposes but belong to different examples, are provided with the same reference signs.

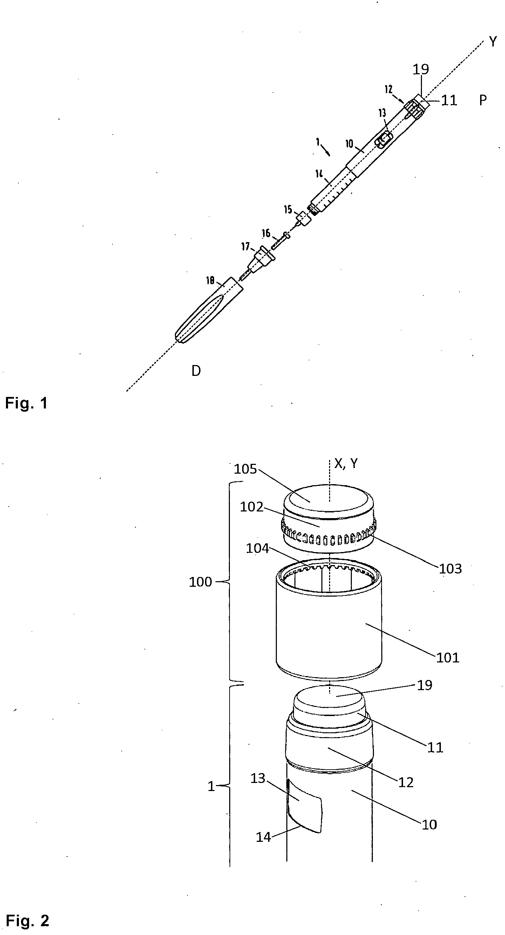

Figure 1 shows an exploded view of an exemplary medicament or drug delivery device 1. The drug delivery device 1 is a pen-type injector comprising a housing 10, i.e. a housing 10 of a drug delivery device 1 or a drug delivery device housing 10, in which a drive mechanism for dose setting and dose dispensing is arranged. The drug delivery device 1 extends from a distal point to a proximal direction P or from a proximal point to a distal direction D along a drug delivery device axis Y of the drug delivery device 1, i.e. a longitudinal axis of the drug delivery device 1. In order to set or dial a dose for delivery a user may rotate or dial a dose dial grip 12 with respect to the housing 10, wherein the dose dial grip 12 is arranged at a proximal end of the housing 10. During dose setting the dose dial grip 12 may perform a helical movement, i.e. a combined axial and rotational movement, or may perform pure rotational movement.

The drive mechanism of the drug delivery device 1 may comprise a plunger, a drive sleeve (partially visible through window 13 in Figure 1), a clutch, a clutch spring, a number sleeve, a last dose nut and so on, which may move during dose setting and/or dose dispensing. Although not all of these components are shown in detail, for example, the drive mechanisms disclosed in EP 1 570 876, EP 2 814 547, US 9,937,294 B2 or WO 2004/078239 A1 represent suitable drive mechanisms for the present disclosure. Although the aforementioned drive mechanisms disclose suitable drive mechanisms for the present disclosure, this does not exclude that although some of these mechanisms do not necessarily generate a noise or a sound, for example a click sound, a corresponding generation, as taught for example in WO 2004/078239, can be advantageous for the occasions described below, but does not have to be. Consequently, reference is made to the documents not for noise reasons but because of a drive mechanism, i.e. a dose dialing mechanism for dose setting and a dose dispensing mechanism for dose delivery. However, other drive mechanisms are also applicable, especially if these drive mechanisms emit a sound, e.g. when setting a dose or when dispensing a dose, in particular a click sound.

Once the dose is set by means of the dose dial grip 12, the user may press a dose button 11 arranged at the proximal end of the drug delivery device 1 in the distal direction D in order to dispense the dose. When pressing the dose button 11, the user applies a force, for example on a proximal end surface 19 of the dose button, directed towards the proximal end of the drug delivery device 1, wherein the force moves the dose button 11 in the distal direction of the pen and parallel to the second longitudinal axis Y. This axial movement of the dose button 11 releases the drive mechanism for example by de-coupling a number sleeve from the drive sleeve, wherein irrespective of which component of the drug delivery device 1 performs a rotational movement during dose delivery, the dose dial grip 12 is coupled to a respective component in order to perform a rotational movement during dose delivery.

The drug delivery device 1 may emit, produce or generate a sound, e.g. a click sound, when the dose dial grip 12 is rotated to set a dose, when the button 11 is pressed to dispense a dose, when components, for example the drive sleeve, inside the drug delivery device move relative to other components, e.g. rotate, when an end position is reached, e.g. an end position during a dose delivery, etc. The sound may be triggered, for example, by a deflected arm, e.g. a deflected clicker arm, inside the drug delivery device 1, which may be deflected due to relative movements of components of the drug delivery device 1.

The exemplary drug delivery device 1 shown in Figure 1 comprises in addition to the dose dial grip 12 and the dose button 11 a display window 13, a needle hub 15 to which a container 14 may be attached and a needle. The set dose may be displayed via the dosage window 13. The container may be filled directly with a drug, for example, insulin or may be configured to receive a cartridge and thus act as a cartridge holder.

The needle may be affixed to the container 14 or the receptacle. During dose dispensing the drug is dispensed through the needle. The needle may be protected by an inner needle cap 16 and/or an outer needle cap 17. In addition, the needle or the inner needle cap 16 may be protected by an outer cap 18.

In order for an electronic add-on module 100 to be functionally attached to a drug delivery device 1, i.e. attached and usable, either the drug delivery device 1 can be adapted to the electronic add-on module 100 or, conversely, the electronic add-on module 100 can be adapted to the drug delivery device 1. In this regard, the electronic add-on module 100 may comprise a coupling portion. The coupling portion may thus be distally arranged and directed towards the drug delivery device, at least when attached.

Figure 2 shows a further schematic example of a drug delivery device 1, wherein an exploded view of an electronic add-on module 100 is arranged above the dose button 11 of the drug delivery device 1. More precisely, above the proximal end surface 19 of the dose button 11, which may be pressed in order to dispense a dose. The schematic example of the drug delivery device 1 only shows the housing 10 together with the proximally arranged dose button 11 and dose dial grip 12. Further, a drive sleeve, which may also be a number sleeve instead, are shown together with the dosage window 13

Dose dialing by rotation of the dose dial grip 12 may cause the dose dial grip 12 together with the dose button 11 to perform a helical, i.e. a combined rotational and axial, movement, wherein the set dose may be displayed in the dosage window 13.

Further, the electronic add-on module 100 comprises a first portion 101 and a second portion 102. Together, the first portion 101 and the second portion 102 and as such outer walls of the first portion 101 and the second portion 102 together form a housing of the electronic add-on module 100. However, the electronic add-on module 100 may also have a one-piece external design, i.e. have only one portion, or have more than two portions, for example three portions. Here, both portions 101 and 102 together provide for a clutch mechanism, which is formed by corresponding clutch teeth 103 on the second portion 102, which may engage in corresponding clutch slots 104 on the first portion 101. When the clutch mechanism is engaged, the first portion 101 and the second portion 102 cannot rotate relative to each other. To be more precise, when the clutch mechanism is engaged, the first portion 101 may not be able to rotate about a longitudinal axis X with respect to the second portion 102.

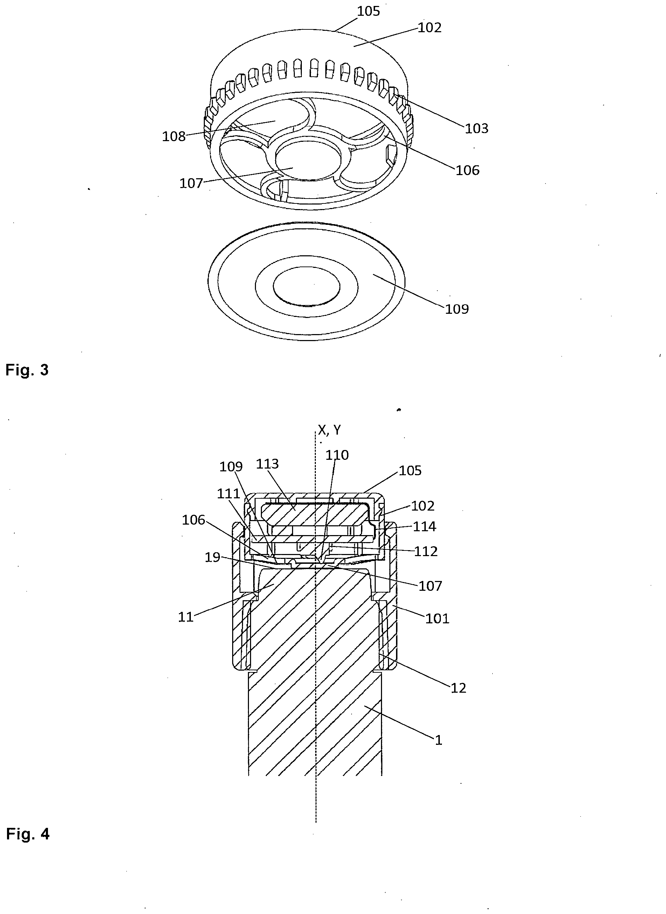

When the electronic add-on module 100 is releasably attached to the drug delivery device 1, for example to the dose dial grip 12 by fastening means for releasable attachment, for example, by interacting mechanical coupling elements or by frictional or elastic engagement, the longitudinal axis X and the drug delivery device axis Y, i.e. the longitudinal axis of the drug delivery device 1, may be in line. Hence, rotation about the longitudinal axis X may also mean rotation about the longitudinal axis Y, for example when the electronic add-on module 100 is attached to the drug delivery device 1 in order to provide an assembly. An assembly of a drug delivery device 1 and an electronic add-on module 100 is for example shown in Figures 4 to 6.

Once attached, a pressure may be applied to a proximal end surface 105 of the electronic add-on module 100 in order to apply pressure to the proximal end surface 19 of the drug delivery device 1, which may cause dose dispensing.

The second portion 102 may comprise a structure as shown in Figure 3, wherein flexible arms 106 are provided, which are connected to a disc 107, for example a central, substantially stiff disc. The disc 107 is connected to the outer wall of the second portion 102 by the flexible arms 106, i.e. in Figure 3 by five flexible arms. The flexible arms 106 are shown with a slight spiral shape, thereby additionally increasing a length of the flexible arms 106, which allows the stiffness to be reduced. Consequently, a corresponding arrangement of flexible arms 106, for example the number of flexible arms 106, the shape or the material of the flexible arms 106, etc., may be used to set whether, when a load is applied to the proximal end surface 105 of the electronic add-on module 100 and when the disc 107 comes into contact with the proximal end surface 19 of the drug delivery device 1, the flexible arms 106 deflect first or the dose button 11 moves first distally and the flexible arms 106 deflect later on, for example, when the dose button 11 cannot be moved further distally because, for example, an end of dose dispensing is reached, i.e. dose delivery has been completed. In one aspect, deflection of the flexible arms 106 may provide for a noise or sound, for example a click sound.

In between the flexible arms 106, there are holes 108 which may be covered or sealed by a membrane 109 in order to fully enclose electronics (described later on) arranged inside the second portion 102. The membrane 109 may thus protect the electronics against water or dirt ingress. The membrane 109 may be a thin flexible adhesive component, such as a structural label or a thin sheet of polymer affixed with adhesive or adhesive tape, affixed to the second portion 102, for example the flexible arms 106.

A corresponding sequence of movement is depicted in Figures 4 to 6, wherein the flexible arms 106 deflect before the dose button 11 is pressed. In the assembly shown in Figure 4, a state is shown prior to any movement. Hence, although disc 107 abuts on the proximal end surface 19 of the dose button 11 no pressure is actively applied, for example by a user, to the proximal end surface 105 and thereby transferred onto the dose button 11.

A switch 110 configured to be actuated in order to activate electronic components of the electronic add-on module 100 is switched off, open or not actuated. Corresponding electronic components may be arranged on a circuit board assembly 111 inside the electronic add-on module 100. Here, the circuit board assembly 111 is arranged inside the second portion 102. Electronic components may for example be chips, switches, wires, sensors, light emitting diodes, displays or the like. For example, an acoustic sensor arrangement 112 may be arranged on the circuit board assembly 111 as shown in Figure 4.

Further, an electric power source 113 is arranged above the circuit board assembly 111 and is electrically connected to the circuit board assembly 111 by an electrically conducting clip 114. The electric power source 113 may be for example a battery or a coin cell. The electric power source 113 may therefore be configured to power the electronic components, for example the acoustic sensor arrangement 113, wherein the switch 110 may control whether the electric power source 113 is powering the electronic components or not.

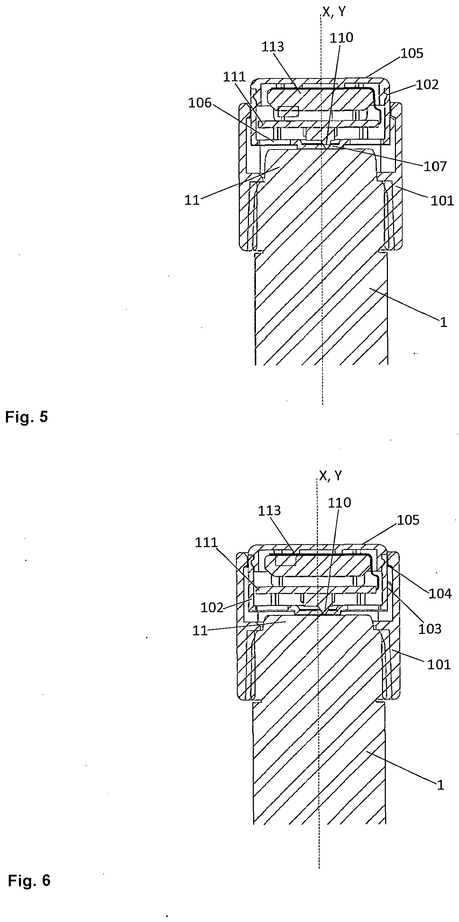

Figure 5 shows an intermediate state, wherein a load on the proximal end face 105 of the electronic add-on module 100 has caused the second portion 102 to move axially along the longitudinal axis X distally relative to the first portion 101, whereby the flexible arms 106 are deflected due to abutment of the disc 107 against the proximal end surface 19 of the dose button 11. Still, the dose button 11 has not been moved distally, however, the movement of the disc 107 has actuated the switch 110 and thus the electronic components may be powered (activated). Hence, in this case a flexible distal surface, provided by the disc 107 and the flexible arms 106, is less stiff and more flexible than drive mechanism so that a force required to move the dose button 11 distally is larger than a force required to deform or deflect the flexible distal surface, i.e. the flexible arms 106. Therefore, the dose button 11 has not been moved, wherein the flexible arms 106 have been deformed with the result of activating, actuating or closing the switch 110. The electronic components may thus be activated.

In this intermediate state it is intended that the neither the clutch mechanism in the module not the clutch mechanism in the pen (which is not shown but present in Figures 4 to 5) have not been disengaged. Consequently, dose dispensing is still prevented.

Figure 6 shows a state in which the dose button 11 has been moved distally and a dose has been dispensed. The depicted state may show the state at first point of dose dispensing starting, but it could equally be at the end of dispense. The full travel to this position is required to start dispensing, with both the clutch in the module and the clutch in the pen disengaged. In other words, in the depicted state, the dose button 11 may be fully depressed and end of dose dispensing may have been reached. The switch 110 may thus still be actuated and the electronic components may still be activated. In addition, the clutch mechanism is now disengaged. Consequently, the electronic components may still be activated at the end of dose dispensing.

However, and as mentioned above, the sequence of movement may also be reversed. In other words, the dose button 11 may first be distally moved and then, for example, close to the end of dose dispensing, the flexible arms 106 are deflected, which may actuate the switch 110 and thus the electronic components. Still, it may be advantageous if the switch 110 is actuated prior to disengagement of the clutch mechanism and, thus, prior to a start of dose dispensing.

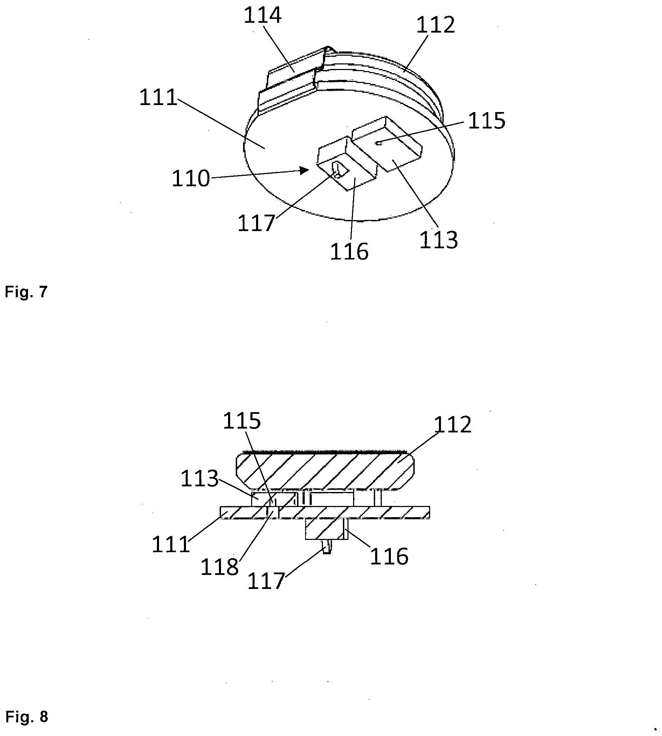

The sequence of movement used, may depend on what is to be recorded or detected. For detection, an acoustic sensor arrangement 113 may be used as shown in Figure 7. The acoustic sensor arrangement 113 may be directly arranged on the circuit board assembly 111 as shown in Figure 7. The acoustic sensor arrangement 113 may be a MEMS (micro-electromechanical systems) acoustic sensor arrangement, for example a capacitive or piezoelectric MEMS acoustic sensor arrangement. The acoustic sensor arrangement 113 may thus comprise a microphone which is MEMS. Considering the assembly shown in Figures 4 to 7, the acoustic sensor arrangement 113 in Figure 7 is arranged on a distal facing surface of the circuit board assembly 111. The acoustic sensor arrangement 113 may comprise a port hole 115, which allows sound waves to enter the acoustic sensor arrangement 113. The sound waves, for example the sound waves of a click sound, may be detected by a sensor, for example a microphone. Here, the port hole 115 is arranged "top-ported", i.e. facing away from the circuit board assembly. However, the port hole 115 may also be arranged "bottom-ported" as will be explained with respect to Figure 8 later on.

Further, the switch 110 is provided by a switch casing 116 into which a switch lever 117 may move, when load is applied. The switch 110 may thus be mechanically moved in order to be operated. When the switch 110 is actuated, the acoustic sensor arrangement 113 may be powered or activated. In other words, the acoustic sensor arrangement 113 may then be in a state in which sounds (sound waves) may be detected. For example, a bias voltage may then be present, which allows a microphone inside the acoustic sensor arrangement 113 to be operated. In addition, further sensor arrangements (not shown) may be present for dose recording, for classifying dose events etc.

In Figure 8, a "bottom-ported" acoustic sensor arrangement 113 is shown. In addition, a through hole 118 is provided in the circuit board assembly 111 in order to allow sound waves to pass via the port hole 115 into the acoustic sensor arrangement 113. The arrangements shown in Figure 7 or 8 may be arranged inside the second portion 102 as shown in Figures 4 to 6, wherein in Figures 4 to 6 the arrangement according to Figure 7 is arranged inside the second portion 102. Consequently, the port hole 115 is directed towards the coupling portion releasably attaching or coupling the electronic add-on module 100 to the drug delivery device.

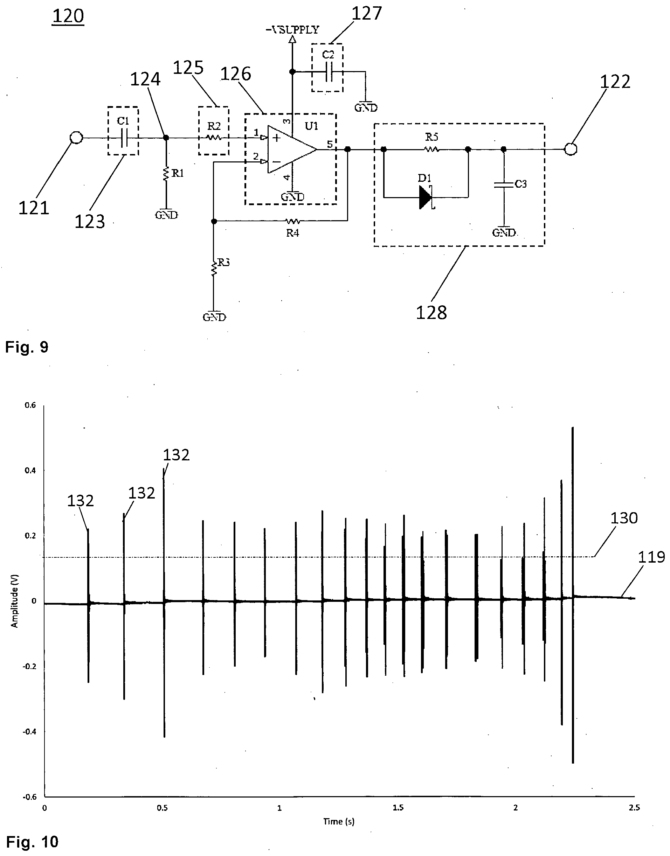

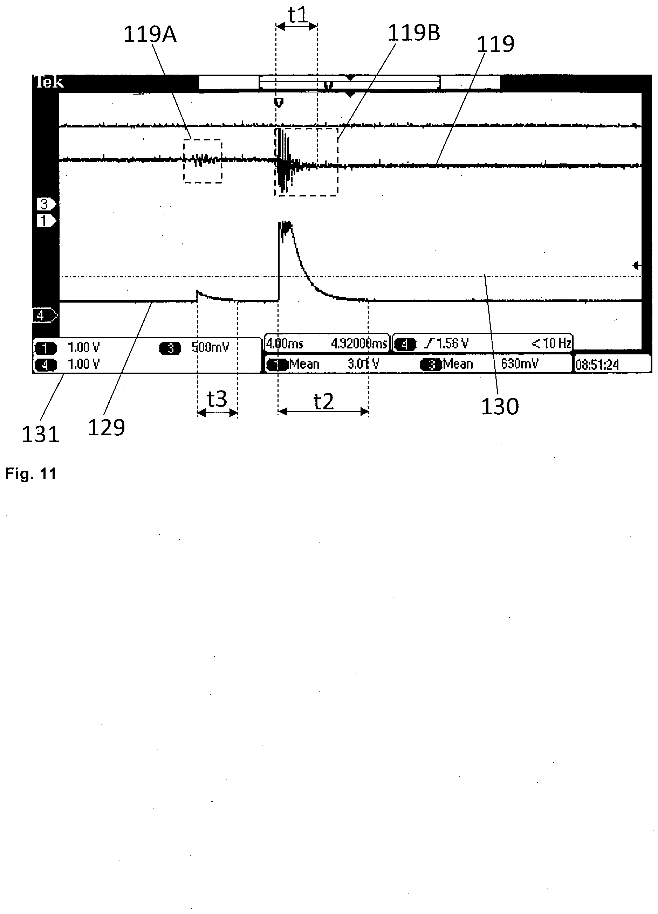

Once a noise or a sound, for example a click sound, is detected by an acoustic sensor arrangement 113, the acoustic sensor arrangement may provide a first output signal 119 as shown in Figure 10 or 11. The first output signal 119 may be a damped sine wave. The first output signal 119 may be in the form of a transient voltage pulse.

In order to allow better processing of the first output signal 119, for example, better detection of dose events, the first output signal 119 may be conditioned by a conditioning circuit 120 as shown in Figure 9. The conditioning circuit 120 comprises a first node 121 connected to the acoustic sensor arrangement 113 output and a second node 122 connected to a processing circuit (not shown).

Further, the conditioning circuit 120 shown here comprises a DC-filter unit 123 configured to remove a DC bias voltage of the acoustic sensor arrangement 113. In the conditioning circuit 120, the DC-filter 123 unit is provided by a capacitor C1. Further, a resistor R1 is provided, which biases the voltage at GND, so that the signal at a third node 124 will be at GND potential when quiescent and have positive or negative voltage components corresponding to a noise or sound, for example a dose click.

Further, the conditioning circuit 120 comprises an electrical impedance unit 125 to limit a current into an amplifier unit 126. The low-pass filter unit 125 is provided by resistor R2. A gain of the amplifier unit 126, which amplifies the first output signal 119 by increasing an amplitude of the first output signal 119, may be set by resistors R3 and R4. In addition, an amplifier-decoupling unit 127 may be provided in order to decouple a power supply to the amplifier unit 126, thereby, for example, reducing transmission of noise (signal noise) present on the electric power source 112 to the conditioning circuit output 120. Here, the amplifier-decoupling unit 127 is provided by decoupling capacitor C2.

Furthermore, an envelope detection circuit 128 configured to modulate the first output signal is provided at the "end" of the conditioning circuit 120, i.e. before the second node 122. The envelope detection circuit 128 may elongate the duration of the first output signal 119 as can be seen in Figure 11, wherein an unconditioned first output signal 119 is depicted above a conditioned first output signal 129, which may be amplified, rectified, smoothed and elongated. The unconditioned first output signal 119 may not have passed a conditioning circuit 120, wherein the conditioning circuit 120 may have passed or may have been modulated or conditioned by the conditioning circuit 120.

The DC-filter unit 123 is located upstream with respect to the electrical impedance unit 125, the amplifier unit 126 and the envelope detection circuit 128. The envelope detection circuit 128 is located downstream with respect to the amplifier unit 126. The amplifier unit 126 is located downstream with respect to the DC-filter unit 123 and the electrical impedance unit 125.

As shown in Figure 9, the envelope detection circuit 120 comprises a diode D1 connected in parallel with a resistor R5. A voltage may thus directly pass through the diode D1 in order to charge a grounded capacitor C3. The capacitor C3 is thus charged quickly which leads a fast response to rising voltages. Still, discharging of the capacitor C3 is performed via the resistor R5. Consequently, the envelope detection circuit 120 allows for a slow response to falling voltages. Therefore, when a high voltage is received, the capacitor C3 may be charged quickly. A lower voltage afterwards may not pass the diode D1 resulting in a delay, stretching or elongating of the signal 119 as can be seen in Figure 11.

The conditioned (and elongated) first output signal may then be used in the processing circuit in order to detect dose events. In Figure 11, a background noise portion 119A of the unconditioned first output signal 119 may show signal of a background noise. Noise portion 119B of the unconditioned first output signal 119 may refer to a relevant noise. In Figure 11, the noise portion 119B refers to a click sound, wherein the signal is used in a processing circuit for detection of dose events. As can be noted, from the conditioned first output signal 129, the portion of the conditioned first output signal 119 referring to the background noise portion 119B does not exceed a threshold value 130. This portion may be disregarded for this reason.

Still further, as the noise portions are relatively short in time, i.e. the prominent amplitudes are only shown for a short duration of time, they are difficult to be processed. Hence, elongating a duration of the first output signal may be advantageous. As can be noted from the conditioned first output signal 129, an unconditioned duration t1 of noise portion 119B of the unconditioned first output signal 119B may thus be elongated to a conditioned duration t2 of the conditioned first output signal 129. Hence, both noises, the non-relevant background noise and the relevant noise, show a signal deflection or voltage pulse, which is elongated when conditioned. The elongated conditioned duration t2 may not only allow for the processing circuit to be slower but may also be used as a criterion for deciding whether the noise is relevant or non-relevant.

For example, the portion of the voltage pulse may only be regarded to refer to a relevant noise when it exceeds a predetermined duration of time. As can be noted from a conditioned duration t3 of the background noise, the duration t3 is shorter than the duration t2. Consequently, the voltage pulse of the conditioned background noise portion 119A may not only be below an amplitude threshold value but may also be shorter in time than a time threshold value, wherein the time threshold value may define a value which is greater than t3 and smaller than t2. The number of times a signal is above the threshold value 130 may be counted to determine the number of set or delivered dose units. Still, confirmation of a dose event, i.e. that a dose dispensing, has actually occurred may be more reliable than counting the number of units dialed or dispensed.

Finally, it should be noted that without conditioning the first output signal, an amplitude of the signal may be fairly small and difficult to be processed, especially when the signal refers to a click sound. As can be noted from a display section 131 in Figure 11, a scale for the different signals shown in Figure 11 is different. In this regard, a value of 500 mV marked with number 3 refers to the unconditioned first output signal 119, wherein a value of 1.00 V marked with number 4 refers to the conditioned first output signal 120. Consequently, when using the same scale, the conditioned first output signal 129 would be even larger compared to the unconditioned first output signal 119, namely the conditioned first output signal 129 would be twice as large as shown here, wherein the unconditioned first output signal 119 would still comprise the same size.

Further, although an unconditioned first output signal 119 as shown in Figure 10 may be used in a processing circuit for detection of dose events, for example by means of threshold values, here an amplitude threshold value 130, this may be quite difficult. The unconditioned first output signal 119 in Figure 10 shows voltage pulse amplitudes that belong to twenty click sounds, wherein each click sound amplitude 132 may be assigned to a single click sound, for example generated during dose setting. The voltage pulse may refer to twenty units dispensed by a FlexPen. The click sound amplitudes 132 may thus be counted. However, the duration of the voltage pulse referring to an individual amplitude of a click sound may only be about a few milliseconds, for example only about 3 ms.

A noise to be detected by an acoustic sensor arrangement 113 as part of an electronic add-on module 100 may thus be generated by a drug delivery device 1 or by the electronic add-on module 100 or by an interaction of the drug delivery device 1 and the electronic add-on module 100. The generated noise may be a click sound, wherein a first output signal of the acoustic sensor arrangement 113 referring to the generated noise may be conditioned in a conditioning circuit 120. The acoustic sensor arrangement 113 may be directly attached to a circuit board assembly 111 of the electronic add-on module 100 and may be "bottom-ported" or "top-ported". The unconditioned first output signal 119 may be conditioned by a conditioning circuit 120 and may afterwards be processed and used for detection in a processing circuit. Conditioning of the unconditioned first output signal 119 may improve detection in the processing circuit. The detection may allow to identify certain dose events, for example a dose dialing event, a dose dispensing event and/or a dose amount, dialed and/or dispensed, based on the first output signal 119, 129 received from the acoustic sensor arrangement 113. Consequently, detection of dose events may be improved or supplemented by using acoustic sounds for dose event detection.

### Reference Numerals

- 1: drug delivery device
- 10: housing (of the drug delivery device)
- 11: dose button
- 12: dose dial grip
- 13: display window
- 14: container
- 15: needle
- 16: inner needle cap
- 17: outer needle cap
- 18: cap
- 19: proximal end surface (of the dose button)

- 100: electronic add-on module
- 101: first portion
- 102: second portion
- 103: clutch teeth
- 104: clutch slots
- 105: proximal end surface (of the electronic add-on module)
- 106: flexible arm
- 107: disc
- 108: hole
- 109: membrane
- 110: switch
- 111: circuit board assembly
- 112: acoustic sensor arrangement
- 113: electric power source
- 114: clip
- 115: port hole
- 116: switch casing
- 117: switch lever
- 118: through hole
- 119: (unconditioned) first output signal
- 119A: background noise portion (of the (unconditioned) first output signal)
- 119B: noise portion (of the (unconditioned) first output signal)
- 120: conditioning circuit
- 121: first node
- 122: second node
- 123: DC-filter unit
- 124: third node
- 125: low-pass filter unit
- 126: amplifier unit
- 127: amplifier-decoupling unit
- 128: envelope detection circuit
- 129: (conditioned) first output signal
- 130: threshold value
- 131: display section
- 132: amplitude of a click sound (of an unconditioned first output signal)

- t1 to t3: (conditioned/unconditioned) durations

- D1: diode
- C1 to C3: capacitors
- R1 to R5: resistors

- D: distal direction
- P: proximal direction
- X: longitudinal axis (of the first portion)
- Y: drug delivery device axis (longitudinal axis of the drug delivery device)

## Claims

1. An electronic add-on module (100) configured for releasable attachment to a drug delivery device (1), the electronic add-on module comprises at least
• a housing (101, 102),
• an electric power source (112) arranged inside the housing,
• a circuit board assembly (111) electrically connected to the electric power source,
• an acoustic sensor arrangement (113) electrically connected to the circuit board assembly, and
• a processing circuit configured to process a first output signal (119, 129) from the acoustic sensor arrangement,
wherein the processing circuit is configured to detect a dose dialing event, a dose dispensing event and/or a dose amount, dialed and/or dispensed, based on the first output signal received from the acoustic sensor arrangement wherein the electronic add-on module comprises a conditioning circuit (120) configured to condition the first output signal (119, 129) of the acoustic sensor arrangement (113) prior to being processed in the processing circuit, and wherein conditioning the first output signal comprises at least one of, several of or all of the following conditioning operations:
• removing a DC voltage;
• limiting a maximum current running in the conditioning circuit;
• amplifying the first output signal by increasing an amplitude of the first output signal; and
modulating the first output signal by elongating a duration the first output signal, rectifying the first output signal and/or smoothing the first output signal.

2. The electronic add-on module (100) according to claim 1, wherein the acoustic sensor arrangement (113) is coupled to the circuit board assembly (111), and wherein the acoustic sensor arrangement comprises a port hole (115) that allows sound waves to enter the acoustic sensor arrangement.

3. The electronic add-on module (100) according to claim 2, wherein the electronic add-on module has a coupling portion for releasable attachment to a drug delivery device (1), and wherein the port hole (115) is directed towards the coupling portion.

4. The electronic add-on module (100) according to any of claims 2 or 3, wherein the circuit board assembly (111), when the electronic add-on module is releasably attached to the drug delivery device (1), is arranged between the drug delivery device and the acoustic sensor arrangement (113), and wherein the circuit board assembly comprises a through hole (118) configured to guide sound waves to the port hole (115) of the acoustic sensor arrangement.

5. The electronic add-on module (100) according to any of the preceding claims, wherein the processing circuit is configured to compare the first output signal (119, 129) from the acoustic sensor arrangement (113) to a threshold value (130), and wherein detection by the processing circuit is triggered based on the comparison with the threshold value.

6. The electronic add-on module (100) according to claim 5, wherein the processing circuit comprises an analog-to-digital converter configured to convert the first output signal (119, 129) to a numerical value, and wherein the processing circuit is configured to compare the numerical value to a numerical threshold value.

7. The electronic add-on module (100) according to any one of the preceding claims, wherein the conditioning circuit comprises at least one of, several of or all of the following components and functionalities:
• a DC-filter unit (123) configured to remove a DC bias voltage of the acoustic sensor arrangement (113);
• a filter electrical impedance unit (125) configured to limit a current into an amplifier unit (126);
• the amplifier unit (126) configured to increase an amplitude of the first output signal (119);
• an amplifier-decoupling unit (127) configured to decouple a power supply to the amplifier unit; and
• an envelope detection circuit (128) configured to modulate the first output signal.

8. The electronic add-on module (100) according to claim 7,
• wherein the envelope detection circuit (128) is connected downstream of the amplifier unit (126) and electrically connected to the processing circuit, and/or
• wherein the DC-filter unit (123) and/or the electrical impedance unit (125) are connected upstream of the amplifier unit (126).

9. The electronic add-on module (100) according to any one of claims 7 or 8, wherein the envelope detection circuit (128) comprises an RC element with a resistor (R5) and a grounded capacitor (C3), and wherein a diode (D1) is connected in parallel with the resistor.

10. The electronic add-on module (100) according to any one of the preceding claims, wherein the electronic add-on module (100) comprises
• a first portion (101) defining an auxiliary dose dial grip and configured to be releasably attached to a dose dial grip (12) of the drug delivery device (1), wherein the first portion has a longitudinal axis (X),
• a second portion (102) coupled to the first portion allowing relative rotational movement about the longitudinal axis (X) and relative axial movement parallel to the longitudinal axis (X) with respect to the first portion, and wherein the second portion defines an auxiliary dose button configured to apply pressure onto the dose button (11) of the drug delivery device when the electronic add-on module (100) is releasably attached to the drug delivery device (1), and
wherein the electronic add-on module additionally comprises a switch (110), wherein the switch is configured to be actuated to activate electronic functionalities of the electronic add-on module, for example to activate the acoustic sensor arrangement (113), and wherein, at least when the electronic add-on module is releasably attached to the drug delivery device, an axial relative movement of the second portion along the longitudinal axis with respect to the first portion actuates the switch.

11. The electronic add-on module (100) according to claim 10, wherein the electronic add-on module (100) additionally comprises a clutch mechanism (103, 104), wherein, when the clutch mechanism is engaged, relative rotational movement about the longitudinal axis (X) between the first portion (101) and the second portion (102) is prevented, wherein, when the clutch mechanism is disengaged, relative rotational movement about the longitudinal axis between the first portion and the second portion is allowed, and wherein, when the electronic add-on module is releasably attached to a drug delivery device (1), the switch (100) is configured to be actuated prior to disengagement of the clutch mechanism.

12. The electronic add-on module (100) according to any one of the preceding claims, wherein the electronic add-on module comprises at least one non-acoustic sensor arrangement, and wherein a second output signal of the at least one non-acoustic sensor arrangement is used to classify a detected dose event into a relevant or a non-relevant dose event.

13. The electronic add-on module (100) according to any one of the preceding claims, wherein the acoustic sensor arrangement (113) is a MEMS acoustic sensor arrangement, for example a capacitive or piezoelectric MEMS acoustic sensor arrangement.

14. An assembly comprising a drug delivery device (1) and an electronic add-on module (100) according to any one of claims 1 to 13, wherein the electronic add-on module is releasably attached to the drug delivery device, wherein the drug delivery device comprises a dose dialing mechanism and/or a dose dispensing mechanism, wherein the actuation of the corresponding mechanism causes a clicking sound, and wherein the electronic add-on module is configured to detect a dose dialing event, a dose dispensing event and/or a dose amount, dialed and/or dispensed, based on a first output signal (119, 129) of an acoustic sensor arrangement (113) resulting from the clicking sound.

## Patentansprüche

1. Elektronisches Zusatzmodul (100), die zur lösbaren Befestigung an einer Arzneimittelabgabevorrichtung (1) gestaltet ist, wobei das elektronische Zusatzmodul zumindest umfasst
• ein Gehäuse (101, 102),
• eine elektrische Energiequelle (112), die im Gehäuse angeordnet ist,
• eine Leiterplattenanordnung (111), die elektrisch mit der elektrischen Energiequelle verbunden ist,
• eine akustische Sensoranordnung (113), die elektrisch mit der Leiterplattenanordnung verbunden ist, und
• eine Verarbeitungsschaltung, die dafür gestaltet ist, ein erstes Ausgangssignal (119, 129) von der akustischen Sensoranordnung zu verarbeiten,
wobei die Verarbeitungsschaltung dafür gestaltet ist, ein Dosiswahlereignis, ein Dosisabgabeereignis und/oder eine gewählte und/oder abgegebene Dosismenge auf der Grundlage des ersten Ausgangssignals, das von der akustischen Sensoranordnung empfangen wird, zu erfassen, wobei das elektronische Zusatzmodul eine Aufbereitungsschaltung (120) umfasst, die dafür gestaltet ist, das erste Ausgangssignal (119, 129) der akustischen Sensoranordnung (113) vor der Verarbeitung in der Verarbeitungsschaltung aufzubereiten, und wobei Aufbereiten des ersten Ausgangssignals zumindest einen, mehrere oder alle der folgenden Aufbereitungsvorgänge umfasst:
• Entfernen einer DC-Spannung;
• Begrenzen eines maximalen Stromes, der in der Aufbereitungsschaltung fließt;
• Verstärken des ersten Ausgangssignals durch Erhöhen einer Amplitude des ersten Ausgangssignals und
Modulieren des ersten Ausgangssignals durch Verlängern einer Dauer des ersten Ausgangssignals, Gleichrichten des ersten Ausgangssignals und/oder Glätten des ersten Ausgangssignals.

2. Elektronisches Zusatzmodul (100) nach Anspruch 1, wobei die akustische Sensoranordnung (113) an die Leiterplattenanordnung (111) gekoppelt ist und wobei die akustische Sensoranordnung eine Durchgangsöffnung (115) umfasst, die ein Eintreten von Schallwellen in die akustische Sensoranordnung ermöglicht.

3. Elektronisches Zusatzmodul (100) nach Anspruch 2, wobei das elektronische Zusatzmodul einen Kopplungsabschnitt zur lösbaren Befestigung an einer Arzneimittelabgabevorrichtung (1) aufweist und wobei die Durchgangsöffnung (115) zum Kopplungsabschnitt hin gerichtet ist.

4. Elektronisches Zusatzmodul (100) nach einem der Ansprüche 2 oder 3, wobei die Leiterplattenanordnung (111), wenn das elektronische Zusatzmodul lösbar an der Arzneimittelabgabevorrichtung (1) befestigt ist, zwischen der Arzneimittelabgabevorrichtung und der akustischen Sensoranordnung (113) angeordnet ist und wobei die Leiterplattenanordnung ein Durchgangsloch (118) umfasst, das dafür gestaltet ist, Schallwellen zu der Durchgangsöffnung (115) der akustischen Sensoranordnung zu leiten.

5. Elektronisches Zusatzmodul (100) nach einem der vorstehenden Ansprüche, wobei die Verarbeitungsschaltung dafür gestaltet ist, das erste Ausgangssignal (119, 129) von der akustischen Sensoranordnung (113) mit einem Schwellenwert (130) zu vergleichen, und wobei eine Erfassung durch die Verarbeitungsschaltung auf der Grundlage des Vergleichs mit dem Schwellenwert ausgelöst wird.

6. Elektronisches Zusatzmodul (100) nach Anspruch 5, wobei die Verarbeitungsschaltung einen Analog-DigitalWandler umfasst, der dafür gestaltet ist, das erste Ausgangssignal (119, 129) in einen numerischen Wert umzuwandeln, und wobei die Verarbeitungsschaltung dafür gestaltet ist, den numerischen Wert mit einem numerischen Schwellenwert zu vergleichen.

7. Elektronisches Zusatzmodul (100) nach einem der vorstehenden Ansprüche, wobei die Aufbereitungsschaltung zumindest eine, mehrere oder alle der folgenden Komponenten und Funktionalitäten umfasst:
• eine DC-Filtereinheit (123), die zum Entfernen einer DC-Vorspannung der akustischen Sensoranordnung (113) gestaltet ist;
• eine elektrische Filterimpedanzeinheit (125), die zum Begrenzen eines Stroms in eine Verstärkereinheit (126) gestaltet ist;
• die Verstärkereinheit (126), die zum Erhöhen einer Amplitude des ersten Ausgangssignals (119) gestaltet ist;
• eine Verstärkerentkopplungseinheit (127), die zum Entkoppeln einer Energieversorgung für die Verstärkereinheit gestaltet ist; und
• eine Hüllkurvenerfassungsschaltung (128), die zum Modulieren des ersten Ausgangssignals gestaltet ist.

8. Elektronisches Zusatzmodul (100) nach Anspruch 7,
• wobei die Hüllkurvenerfassungsschaltung (128) der Verstärkereinheit (126) nachgeschaltet und elektrisch mit der Verarbeitungsschaltung verbunden ist und/oder
• wobei die DC-Filtereinheit (123) und/oder die elektrische Impedanzeinheit (125) der Verstärkereinheit (126) vorgeschaltet sind.

9. Elektronisches Zusatzmodul (100) nach einem der Ansprüche 7 oder 8, wobei die Hüllkurvenerfassungsschaltung (128) ein RC-Element mit einem Widerstand (R5) und einem geerdeten Kondensator (C3) umfasst und wobei eine Diode (D1) parallel zum Widerstand geschaltet ist.

10. Elektronisches Zusatzmodul (100) nach einem der vorstehenden Ansprüche, wobei das elektronische Zusatzmodul (100) umfasst
• einen ersten Abschnitt (101), der einen Hilfsdosiswahlgriff definiert und dafür gestaltet ist, lösbar an einem Dosiswahlgriff (12) der Arzneimittelabgabevorrichtung (1) befestigt zu werden, wobei der erste Abschnitt eine Längsachse (X) aufweist,
• einen zweiten Abschnitt (102), der an den ersten Abschnitt gekoppelt ist und eine relative Drehbewegung um die Längsachse (X) und eine relative Axialbewegung parallel zur Längsachse (X) bezogen auf den ersten Abschnitt ermöglicht, und wobei der zweite Abschnitt einen Hilfsdosisknopf definiert, der dafür gestaltet ist, Druck auf den Dosisknopf (11) der Arzneimittelabgabevorrichtung auszuüben, wenn das elektronische Zusatzmodul (100) lösbar an der Arzneimittelabgabevorrichtung (1) befestigt ist, und
wobei das elektronische Zusatzmodul zusätzlich einen Schalter (110) umfasst, wobei der Schalter dafür gestaltet ist, betätigt zu werden, um elektronische Funktionalitäten des elektronischen Zusatzmoduls zu aktivieren, beispielsweise um die akustische Sensoranordnung (113) zu aktivieren, und wobei, zumindest wenn das elektronische Zusatzmodul lösbar an der Arzneimittelabgabevorrichtung befestigt ist, eine axiale Relativbewegung des zweiten Abschnitts entlang der Längsachse bezogen auf den ersten Abschnitt den Schalter betätigt.

11. Elektronisches Zusatzmodul (100) nach Anspruch 10, wobei das elektronische Zusatzmodul (100) zusätzlich einen Kupplungsmechanismus (103, 104) umfasst, wobei, wenn der Kupplungsmechanismus in Eingriff ist, eine relative Drehbewegung um die Längsachse (X) zwischen dem ersten Abschnitt (101) und dem zweiten Abschnitt (102) verhindert wird, wobei, wenn der Kupplungsmechanismus gelöst ist, eine relative Drehbewegung um die Längsachse zwischen dem ersten Abschnitt und dem zweiten Abschnitt möglich ist, und wobei, wenn das elektronische Zusatzmodul lösbar an einer Arzneimittelabgabevorrichtung (1) befestigt ist, der Schalter (100) dafür gestaltet ist, vor einem Lösen des Kupplungsmechanismus betätigt zu werden.

12. Elektronisches Zusatzmodul (100) nach einem der vorstehenden Ansprüche, wobei das elektronische Zusatzmodul zumindest eine nicht-akustische Sensoranordnung umfasst und wobei ein zweites Ausgangssignal der zumindest einen nicht-akustischen Sensoranordnung verwendet wird, um ein erfasstes Dosisereignis als ein relevantes oder ein nicht relevantes Dosisereignis einzustufen.

13. Elektronisches Zusatzmodul (100) nach einem der vorstehenden Ansprüche, wobei die akustische Sensoranordnung (113) eine akustische MEMS-Sensoranordnung, beispielsweise eine kapazitive oder piezoelektrische akustische MEMS-Sensoranordnung, ist.

14. Anordnung, umfassend eine Arzneimittelabgabevorrichtung (1) und ein elektronisches Zusatzmodul (100) nach einem der Ansprüche 1 bis 13, wobei das elektronische Zusatzmodul lösbar an der Arzneimittelabgabevorrichtung befestigt ist, wobei die Arzneimittelabgabevorrichtung einen Dosiswahlmechanismus und/oder einen Dosisabgabemechanismus umfasst, wobei die Betätigung des entsprechenden Mechanismus ein Klickgeräusch verursacht und wobei das elektronische Zusatzmodul dafür gestaltet ist, ein Dosiswahlereignis, ein Dosisabgabeereignis und/oder eine gewählte und/oder abgegebene Dosismenge auf der Grundlage eines ersten Ausgangssignals (119, 129) einer akustischen Sensoranordnung (113), das aus dem Klickgeräusch folgt, zu erfassen.

## Revendications

1. Module complémentaire électronique (100) configuré pour être fixé de manière amovible à un dispositif d'administration de médicament (1), le module complémentaire électronique comprenant au moins
• un boîtier (101, 102),
• une source d'énergie électrique (112) agencée à l'intérieur du boîtier,
• un ensemble carte de circuit imprimé (111) connecté électriquement à la source d'énergie électrique,
• un agencement de capteur acoustique (113) connecté électriquement à l'ensemble carte de circuit imprimé, et
• un circuit de traitement configuré pour traiter un premier signal de sortie (119, 129) provenant de l'agencement de capteur acoustique,
dans lequel le circuit de traitement est configuré pour détecter un événement de sélection de dose, un événement de distribution de dose et/ou une quantité de dose, sélectionnée et/ou distribuée, sur la base du premier signal de sortie reçu de l'agencement de capteur acoustique, dans lequel le module complémentaire électronique comprend un circuit de conditionnement (120) configuré pour conditionner le premier signal de sortie (119, 129) de l'agencement de capteur acoustique (113) avant un traitement dans le circuit de traitement, et dans lequel le conditionnement du premier signal de sortie comprend au moins une, plusieurs, ou toutes les opérations de conditionnement suivantes :
• élimination d'une tension continue ;
• limitation d'un courant maximal circulant dans le circuit de conditionnement ;
• amplification du premier signal de sortie en augmentant une amplitude du premier signal de sortie ; et
modulation du premier signal de sortie par allongement d'une durée du premier signal de sortie, redressement du premier signal de sortie et/ou lissage du premier signal de sortie.

2. Module complémentaire électronique (100) selon la revendication 1, dans lequel l'agencement de capteur acoustique (113) est couplé à l'ensemble carte de circuit imprimé (111), et dans lequel l'agencement de capteur acoustique comprend un trou de port (115) qui permet à des ondes sonores d'entrer dans l'agencement de capteur acoustique.

3. Module complémentaire électronique (100) selon la revendication 2, dans lequel le module complémentaire électronique a une partie d'accouplement pour une fixation amovible à un dispositif d'administration de médicament (1), et dans lequel le trou de port (115) est orienté vers la partie d'accouplement.

4. Module complémentaire électronique (100) selon l'une quelconque des revendications 2 et 3, dans lequel l'ensemble carte de circuit imprimé (111), lorsque le module complémentaire électronique est fixé de manière amovible au dispositif d'administration de médicament (1), est agencé entre le dispositif d'administration de médicament et l'agencement de capteur acoustique (113), et dans lequel l'ensemble carte de circuit imprimé comprend un trou traversant (118) configuré pour guider les ondes sonores vers le trou de port (115) de l'agencement de capteur acoustique.

5. Module complémentaire électronique (100) selon l'une quelconque des revendications précédentes, dans lequel le circuit de traitement est configuré pour comparer le premier signal de sortie (119, 129) provenant de l'agencement de capteur acoustique (113) à une valeur seuil (130), et dans lequel la détection par le circuit de traitement est déclenchée sur la base de la comparaison avec la valeur seuil.

6. Module complémentaire électronique (100) selon la revendication 5, dans lequel le circuit de traitement comprend un convertisseur analogique-numérique configuré pour convertir le premier signal de sortie (119, 129) en une valeur numérique, et dans lequel le circuit de traitement est configuré pour comparer la valeur numérique à une valeur numérique seuil.

7. Module complémentaire électronique (100) selon l'une quelconque des revendications précédentes, dans lequel le circuit de conditionnement comprend au moins l'un, plusieurs ou la totalité des composants et fonctionnalités suivants :
• une unité de filtre CC (123) configurée pour éliminer une tension de polarisation CC de l'agencement de capteur acoustique (113) ;
• une unité d'impédance électrique de filtre (125) configurée pour limiter un courant dans une unité d'amplificateur (126) ;
• l'unité d'amplificateur (126) configurée pour augmenter une amplitude du premier signal de sortie (119) ;
• une unité de découplage d'amplificateur (127) configurée pour découpler une alimentation électrique de l'unité d'amplificateur ; et
• un circuit de détection d'enveloppe (128) configuré pour moduler le premier signal de sortie.

8. Module complémentaire électronique (100) selon la revendication 7,
• dans lequel le circuit de détection d'enveloppe (128) est connecté en aval de l'unité d'amplificateur (126) et connecté électriquement au circuit de traitement, et/ou
• dans lequel l'unité de filtre CC (123) et/ou l'unité d'impédance électrique (125) sont connectées en amont de l'unité d'amplificateur (126).

9. Module complémentaire électronique (100) selon l'une quelconque des revendications 7 et 8, dans lequel le circuit de détection d'enveloppe (128) comprend un élément RC avec une résistance (R5) et un condensateur mis à la masse (C3), et dans lequel une diode (D1) est connectée en parallèle avec la résistance.

10. Module complémentaire électronique (100) selon l'une quelconque des revendications précédentes, dans lequel le module complémentaire électronique (100) comprend
• une première partie (101) définissant une partie de préhension auxiliaire de cadran de dose et configurée pour être fixée de manière amovible à une partie de préhension de cadran de dose (12) du dispositif d'administration de médicament (1), dans lequel la première partie a un axe longitudinal (X),
• une seconde partie (102) accouplée à la première partie permettant un mouvement de rotation relatif autour de l'axe longitudinal (X) et un mouvement axial relatif parallèle à l'axe longitudinal (X) par rapport à la première partie, et dans lequel la seconde partie définit un bouton de dose auxiliaire configuré pour appliquer une pression sur le bouton de dose (11) du dispositif d'administration de médicament lorsque le module complémentaire électronique (100) est fixé de manière amovible au dispositif d'administration de médicament (1), et
dans lequel le module complémentaire électronique comprend en outre un commutateur (110), dans lequel le commutateur est configuré pour être actionné pour activer des fonctionnalités électroniques du module complémentaire électronique, par exemple pour activer l'agencement de capteur acoustique (113), et dans lequel, au moins lorsque le module complémentaire électronique est fixé de manière amovible au dispositif d'administration de médicament, un mouvement relatif axial de la seconde partie le long de l'axe longitudinal par rapport à la première partie actionne le commutateur.

11. Module complémentaire électronique (100) selon la revendication 10, dans lequel le module complémentaire électronique (100) comprend en outre un mécanisme d'embrayage (103, 104), dans lequel, lorsque le mécanisme d'embrayage est en prise, un mouvement de rotation relatif autour de l'axe longitudinal (X) entre la première partie (101) et la seconde partie (102) est empêché, dans lequel, lorsque le mécanisme d'embrayage est libéré, un mouvement de rotation relatif autour de l'axe longitudinal entre la première partie et la seconde partie est autorisé, et dans lequel, lorsque le module complémentaire électronique est fixé de manière amovible à un dispositif d'administration de médicament (1), le commutateur (100) est configuré pour être actionné avant la libération du mécanisme d'embrayage.

12. Module complémentaire électronique (100) selon l'une quelconque des revendications précédentes, dans lequel le module complémentaire électronique comprend au moins un agencement de capteur non acoustique, et dans lequel un second signal de sortie de l'au moins un agencement de capteur non acoustique est utilisé pour classer un événement de dose détecté en un événement de dose pertinent ou non pertinent.

13. Module complémentaire électronique (100) selon l'une quelconque des revendications précédentes, dans lequel l'agencement de capteur acoustique (113) est un agencement de capteur acoustique MEMS, par exemple un agencement de capteur acoustique MEMS capacitif ou piézoélectrique.

14. Ensemble comprenant un dispositif d'administration de médicament (1) et un module complémentaire électronique (100) selon l'une quelconque des revendications 1 à 13, dans lequel le module complémentaire électronique est fixé de manière amovible au dispositif d'administration de médicament, dans lequel le dispositif d'administration de médicament comprend un mécanisme de sélection de dose et/ou un mécanisme de distribution de dose, dans lequel l'actionnement du mécanisme correspondant provoque un bruit de clic, et dans lequel le module complémentaire électronique est configuré pour détecter un événement de sélection de dose, un événement de distribution de dose et/ou une quantité de dose, sélectionnée et/ou distribuée, sur la base d'un premier signal de sortie (119, 129) d'un agencement de capteur acoustique (113) résultant du bruit de clic.
